# EUROPEAN PATENT APPLICATION

(11) **EP 4 501 913 A1**
(43) Date of publication of application: **05.02.2025**
(21) Application number: 23780905.8
(22) Date of filing: 30.03.2023
(51) Int. Cl.: C07D 217/24, A61K 31/472, A61P 21/02

(54) **MUSCLE RELAXANT**

(30) Priority: 30.03.2022 JP 2022056322
(71) Applicant: Sunaga, Hiroshi, Tokyo 105-8461 (JP)
(72) Inventor: Sunaga, Hiroshi, Tokyo 105-8461 (JP)
(74) Representative: Gill Jennings & Every LLP
(86) International application number: PCT/JP2023/013247
(87) International publication number: WO 2023/190897

(57) **Abstract**

Provided is a novel compound that can be used as a neuromuscular blocking agent, and specifically is a compound represented by the general formula (I) or a pharmacologically acceptable salt thereof, in which X₁, X₂, X₃, and X₄ each independently represent hydrogen or a C₁₋₃ alkoxy group, and at least two of X₁, X₂, X₃, and X₄ are C₁₋₃ alkoxy groups; Y₁, Y₂, Y₃, Y₄, and Y₅ each independently represent hydrogen or a C₁₋₃ alkoxy group, and at least two of Y₁, Y₂, Y₃, Y₄, and Y₅ are C₁₋₃ alkoxy groups; n is 0 or 1; and L represents a linker with a main chain of 5 to 20 atoms.

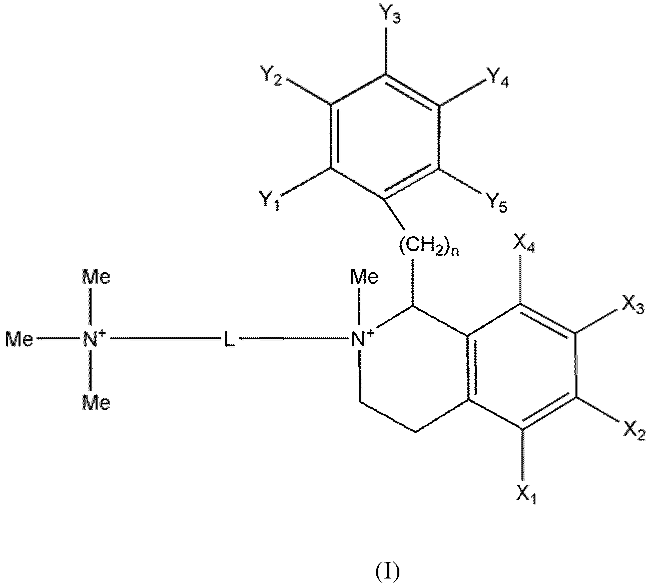

## Description

### TECHNICAL FIELD

The present invention relates to novel compounds and pharmaceuticals comprising them as active ingredients that exhibit neuromuscular blocking effects.

### BACKGROUND ART

Neuromuscular blocking agents are used to facilitate tracheal intubation required in general anesthesia or to prevent body movement during surgery; however, their effects are no longer needed once the surgery is completed. If the muscle paralysis persists, it can prevent the patient from being weaned from ventilation, delay their discharge from the operating room, and negatively impact the efficient management of the operating room. Residual neuromuscular block also has a negative impact on the occurrence of postoperative pulmonary complications. Therefore, neuromuscular blocking agents with rapid offset and short duration of action are needed in clinical practice.

Currently, the neuromuscular blocking agents used in clinical anesthesia include depolarizing neuromuscular blocking agents (succinylcholine) and nondepolarizing neuromuscular blocking agents (rocuronium). Nondepolarizing neuromuscular blocking agents (rocuronium) can have their offset of action shortened with the administration of an antagonist (sugammadex), but they have the drawback of requiring complex management. Additionally, the antagonist (sugammadex) can often cause side effects such as anaphylaxis. Therefore, succinylcholine, which can be expected to achieve a rapid offset without the need to administer an antagonist, is considered simpler and easier to use.

Succinylcholine is a drug that has been in use since it began clinical application around 1950, but no neuromuscular blocking agents that have a faster offset of action than succinylcholine during spontaneous recovery (without administration of antagonists) have been developed. However, the duration of action at clinical doses is reported to be approximately 10 minutes (Non-Patent Document 1), which is not satisfactory enough for situations, for example, requiring urgent recovery from muscle paralysis, such as in cases of inability to ventilate or intubate.

### PRIOR ART REFERENCES

### NON-PATENT DOCUMENT

Non-Patent Document 1: Schmartz D, Chenard L, Baumann C, Fuchs-Buder T., A modified train-of-four ratio to assess recovery from depolarizing neuromuscular blockade after succinylcholine, a prospective observational study. J Clin Monit Comput. 2021 Oct; 35(5):1133-1138. doi: 10.1007/s10877-020-00560-5.

### SUMMARY OF THE INVENTION

### PROBLEMS TO BE SOLVED BY THE INVENTION

An object of the present invention is to provide a compound that can be used as a neuromuscular blocking agent having a short duration of action and rapid loss of action.

### MEANS FOR SOLVING THE PROBLEMS

In order to solve the above problems, the present inventors have intensively studied to find that a compound represented by the general formula (I) exerts an excellent effect as a neuromuscular blocking agent with a short duration of action, thereby completing the present invention.

Thus, one aspect of the present invention relates to a compound represented by the general formula (I) or a pharmacologically acceptable salt thereof, and a pharmaceutical composition comprising it as an active ingredient, used as a neuromuscular blocking agent.

### EFFECT OF THE INVENTION

The present invention provides a group of compounds characterized by a short duration of neuromuscular blocking effect. More specifically, the compounds of the present invention have a shorter duration of action than succinylcholine that has the shortest duration of action among neuromuscular blocking agents currently in clinical use. Therefore, there is no need for precise adjustments in dosage or administration rate considering the duration of action, making its clinical management easier. In addition, increasing the dosage can accelerate the onset of action, and continuous administration can maintain a stable and deep neuromuscular blockade. The invention can also make it possible to deal with situations that require urgent recovery from neuromuscular blockade, such as ventilation and intubation difficulties. Furthermore, the invention can avoid the disadvantages of long-lasting muscle paralysis and eliminate the need for antagonists to accelerate the offset of the action, thereby reducing the risk of anaphylactic shock and enabling re-administration during reoperation.

### BRIEF DESCRIPTION OF THE DRAWINGS

FIG. 1 shows graphs comparing the duration of action of JH013 and succinylcholine administered at doses inducing 95-99.9% neuromuscular block (ED95-99.9) in rats.
FIG. 2 shows graphs comparing the duration of action of JH014 and succinylcholine administered at doses inducing 95-99.9% neuromuscular block (ED95-99.9) in rats.

### DETAILED DESCRIPTION OF THE INVENTION

A compound of the present invention is represented by the general formula (I):

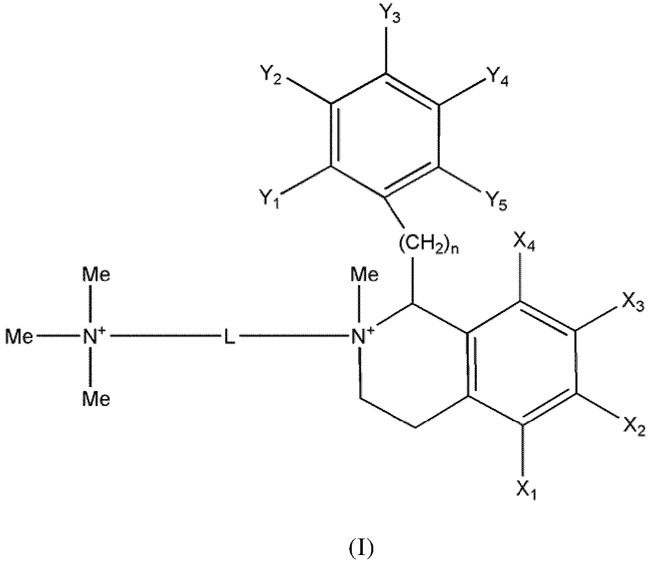

In the general formula (I), X₁, X₂, X₃, and X₄ each independently represent hydrogen or a C₁₋₃ alkoxy group; at least two of X₁, X₂, X₃, and X₄ are C₁₋₃ alkoxy groups, preferably two of X₁, X₂, X₃, and X₄ are C₁₋₃ alkoxy groups, and more preferably X₂ and X₃ are C₁₋₃ alkoxy groups, and X₁ and X₄ are hydrogen. The C₁₋₃ alkoxy group is preferably a methoxy group.

Y₁, Y₂, Y₃, Y₄, and Y₅ each independently represent hydrogen or a C₁₋₃ alkoxy group; at least two of Y₁, Y₂, Y₃, Y₄, and Y₅ are C₁₋₃ alkoxy groups, and preferably two of Y₁, Y₂, Y₃, Y₄, and Y₅ are C₁₋₃ alkoxy groups. More preferably, Y₃ and either Y₂ or Y₄ of Y₁, Y₂, Y₃, Y₄, and Y₅ are C₁₋₃ alkoxy, and the rest is hydrogen. The C₁₋₃ alkoxy group is preferably a methoxy group.

n is 0 or 1, and preferably is 1.

L represents a linker with a main chain of 5 to 20 atoms, preferably a linker with a main chain of 5 to 16 atoms, more preferably a linker with a main chain of 7 to 16 atoms, still more preferably a linker with a main chain of 8 to 13 atoms, still more preferably a linker with a main chain of 9 to 12 atoms, and particularly preferably a linker with a main chain of 10 to 11 atoms.

Preferably, L is a divalent alkylene or alkenylene group having a main chain of 5 to 20, 5 to 16, 7 to 16, 8 to 13, 9 to 12, or 10 to 11 carbon, wherein one or more (preferably two or more, more preferably 2 to 4, still more preferably two) -CH₂- are replaced by -O- or -O-C(=O)- or -C(=O)-O-. In this divalent group, one or more hydrogen may be replaced by halogen.

L is selected from the following general formulae (II) to (V), for example:

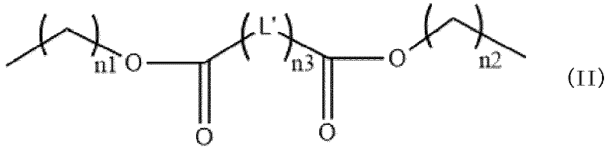

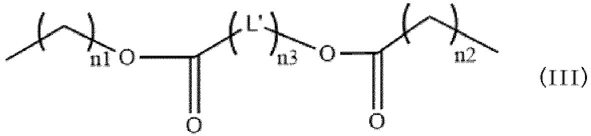

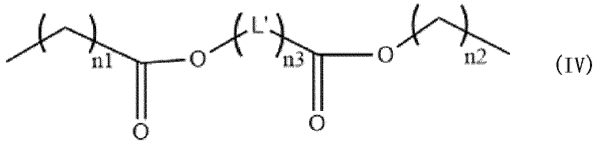

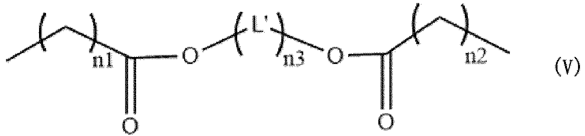

In the general formulae (II) to (V), -(L')ₙ₃- is a C₁₋₆ alkylene or alkenylene group, wherein one or more hydrogen may be replaced by halogen (e.g., Cl, F, or Br); n1 is an integer of 1 to 5 (preferably 1 to 3), and n2 is an integer of 1 to 5 (preferably 1 to 3).

In other aspects, L is represented, for example, by the general formula (VI): 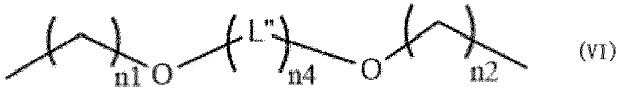 wherein -(L")ₙ₄- is a C₁₋₆ alkylene or alkenylene group, wherein one or more hydrogen may be replaced by halogen, n1 is an integer of 1 to 5 (preferably 1 to 3), and n2 is an integer of 1 to 5 (preferably 1 to 3).

Examples of the compound of the general formula (I) include the following compounds:

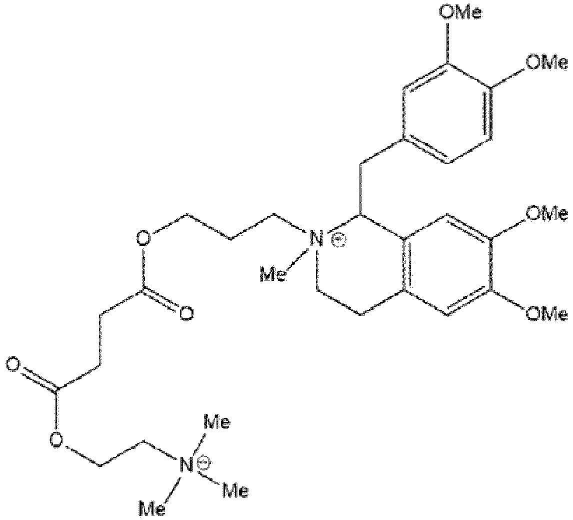

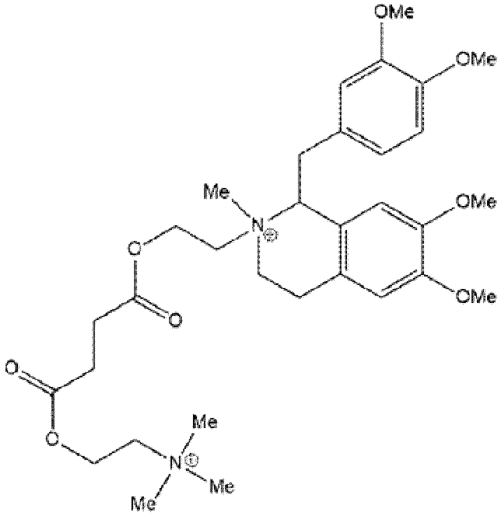

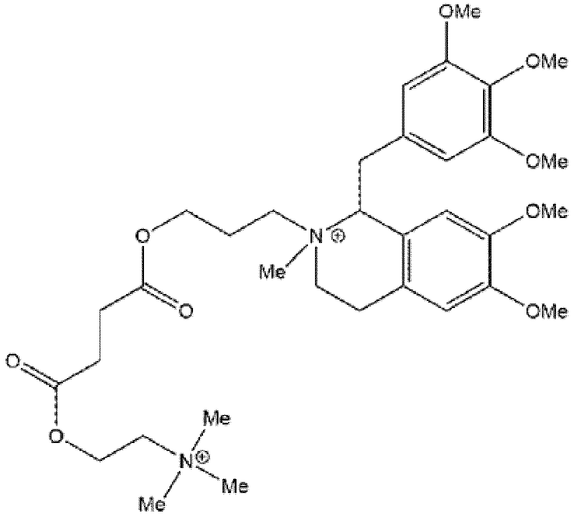

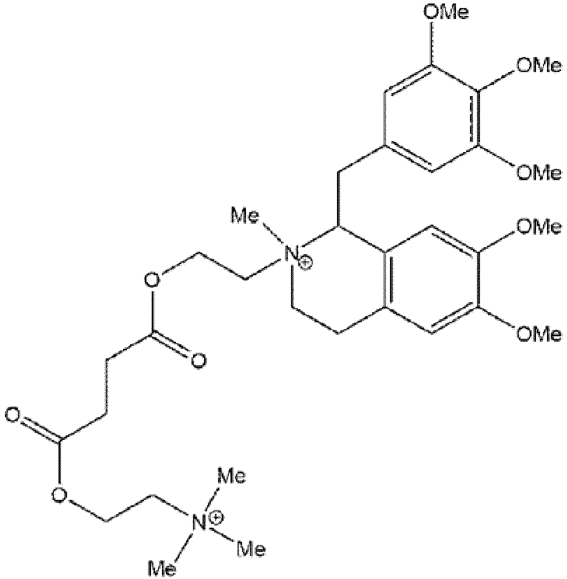

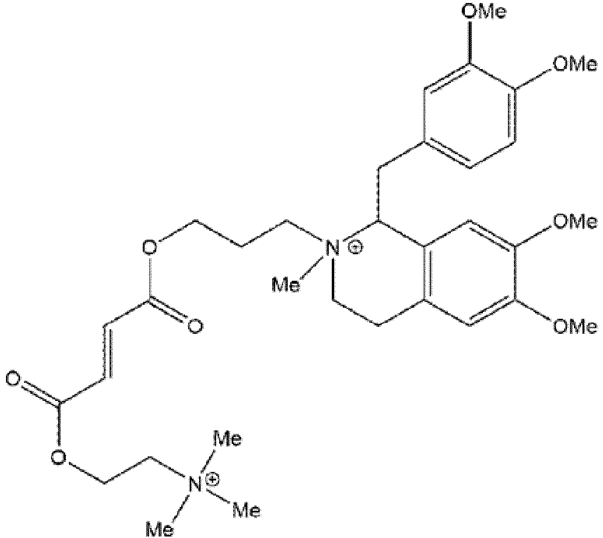

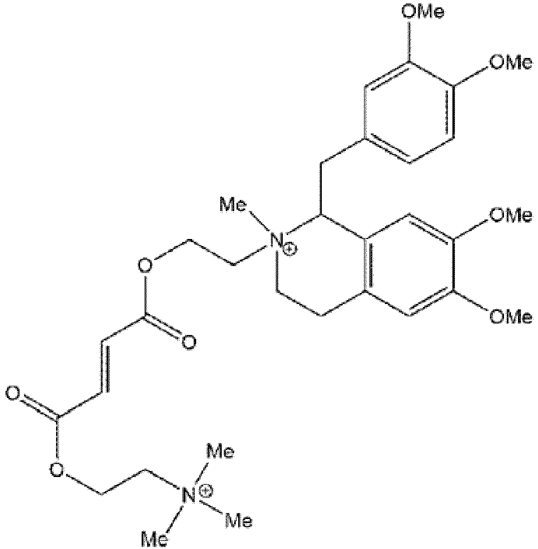

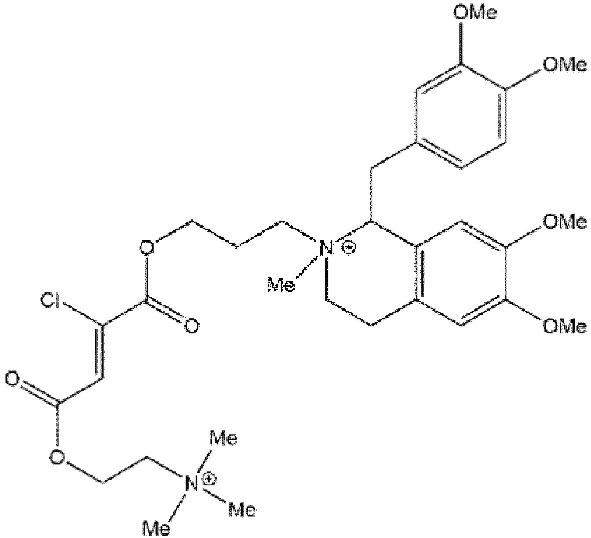

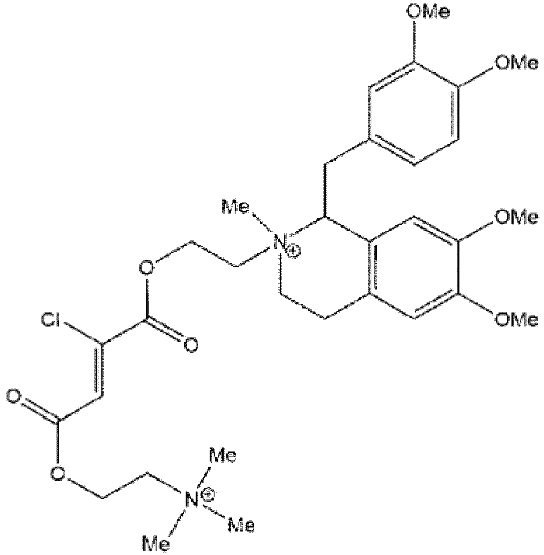

The compound of the general formula (I) can be synthesized by the method as described in the examples.

Salts of the compound of the general formula (I) are not particularly limited as long as they are pharmacologically acceptable salts, and include, for example, acid addition salts. The acid addition salts include salts with inorganic acids such as hydrochloric acid, sulfuric acid, nitric acid, and phosphoric acid, and salts with organic acids such as acetic acid, malic acid, succinic acid, tartaric acid, citric acid, fumaric acid, oxalic acid, and tosic acid, and salt with chlorine.

The compound of the general formula (I), when the molecule has an asymmetric carbon atom, can exist as multiple stereoisomers (i.e., diastereomers or optical isomers) based on the asymmetric carbon atom, any one or a mixture of which stereoisomers are included in the present invention.

The compounds of general formula (I) may also include cis- and trans-isomers as geometric isomers, and when the molecules have an axial chirality, they may further include isomers based on the axial chirality. Any one isomer within these isomers or a mixture of these isomers are included.

The compound of the present invention can be a pharmaceutical composition comprising a compound of the general formula (I) or a salt thereof as an active ingredient and a pharmacologically acceptable carrier.

Examples of the pharmacologically acceptable carrier include excipients, binders, disintegrants, and lubricants that can be commonly used, as well as various carriers, stabilizers, surfactants, plasticizers, lubricating agent, solubilizing agents, reducing agents, buffer agents, sweetening agents, bases, adsorbents, taste masking agents, suspending agents, antioxidants, glossing agents, coating agents, film coatings, wetting agents, humidity regulators, fillers, antifoams, cooling agents, coloring agents, flavoring agents, flavors, sugar coatings, isotonizing agents, softening agents, emulsifiers, thickening agents, viscosity modifiers, foaming agents, pH adjusters, diluting agents, dispersants, disintegrants, disintegration retarders, aromatics, desiccants, preservatives, preservatives, solubilizers, dissolution aids, solvents, glidants, antistatic agents, bulking agents, moisturizers, and humectants.

Examples of the dosage form of the pharmaceuticals include, but not particularly limited to, liquid agents, injections, inhalants, external preparations, tablets, capsules, and emulsions. The route of administration is not particularly limited, and the pharmaceuticals can be formulated as parenteral preparations, for example, for subcutaneous, intramuscular, intrarectal, or intranasal administration.

The amount of the compound of the general formula (I) or a salt thereof as an active ingredient in the pharmaceutical composition is not particularly restricted as long as it is an amount effective to achieve the pharmaceutical effect. The amount is generally varies depending on the dosage form, and the amount of the active ingredient can be set, for example, within the range of about 0.01% by mass to about 99.9 % by mass to achieve the desired dose.

The pharmaceutical comprising the compound of the general formula (I) or a salt thereof of the present invention can be suitably used as a neuromuscular blocking agent.

Neuromuscular blocking agents are classified into central neuromuscular blocking agents and peripheral neuromuscular blocking agents based on their site of action. Clinically, for example, neuromuscular blocking agents can be used to treat spastic paralysis, improve the myotonic state, paralyse muscles during anesthesia or tracheal intubation, and for malignant syndrome (dantrolene), and blepharospasm (botulinum toxin).

The desirable dosage of the compound of the general formula (I) or a salt thereof varies, for example, depending on the target of administration, dosage form, method of administration, and duration of administration. To achieve the desired effect, generally 0.5 to 1,000 mg, preferably 1 to 500 mg can be administered for adults.

Other aspects of the present invention include, for example, the following:
a method for neuromuscular blockade comprising administering a compound of general formula (I) or a salt thereof to a subject in need thereof;
a method for treating spastic paralysis, comprising administering a compound of general formula (I) or a salt thereof to a subject in need thereof;
a method for ameliorating a myotonic state, comprising administering a compound of general formula (I) or a salt thereof to a subject in need thereof;
a method for treating a malignant syndrome, comprising administering a compound of general formula (I) or a salt thereof to a subject in need thereof; and
a method for treating blepharospasm, comprising administering a compound of general formula (I) or a salt thereof to a subject in need thereof.

Other aspects of the present invention also include, for example, the following:
a compound of the general formula (I) or a salt thereof, for use as a neuromuscular blocking agent;
a compound of the general formula (I) or a salt thereof, for use in treating spastic paralysis;
a compound of the general formula (I) or a salt thereof, for use in ameliorating a myotonic state;
a compound of the general formula (I) or a salt thereof, for use in treating a malignant syndrome; and
a compound of the general formula (I) or a salt thereof, for used in treating blepharospasm.

Other aspects of the present invention further include, for example, the following:
use of a compound of the general formula (I) or a salt thereof in the manufacture of a neuromuscular blocking agent;
use of a compound of the general formula (I) or a salt thereof in the manufacture of a pharmaceutical for treating spastic paralysis;
use of a compound of the general formula (I) or a salt thereof in the manufacture of a pharmaceutical for ameliorating a myotonic state;
use of a compound of the general formula (I) or a salt thereof in the manufacture of a pharmaceutical for treating a malignant syndrome; and
use of a compound of the general formula (I) or a salt thereof in the manufacture of a pharmaceutical for treating blepharospasm.

### EXAMPLES

The present invention will be described in detail below with reference to Examples, but is not limited to the aspects of the following Examples.

### Scheme of Synthesis of Compound JH013

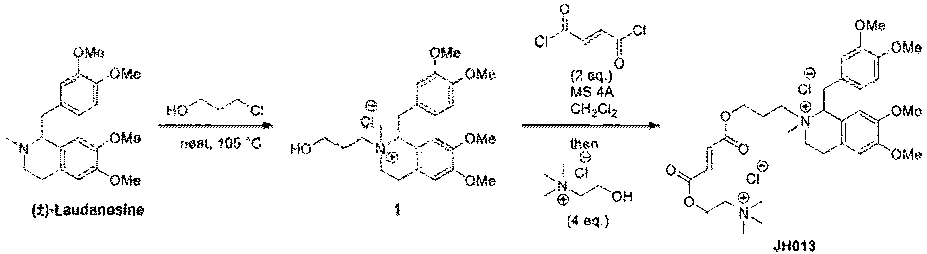

### <Synthesis of Intermediate 1>

To (±)-Laudanosine (2.86 g, 8.00 mmol) was added 3-chloro-1-propanol (3.32 mL, 40.0 mmol), then the mixture was heated at 105°C for 14 hours. After leaving the mixture to cool, acetonitrile (5 mL) was added to completely dissolve the precipitated target substance. Then, the reaction solution was added dropwise to diisopropyl ether (150 mL), and the precipitated solids were collected through filtration. The same operation was repeated twice for the resulting solids received. The resulting crude product (3.71 g) was purified by medium-pressure column chromatography on silica gel (100 g of diol silica gel, methylene chloride/methanol = 100/0 to 95/5) to obtain Intermediate 1 (1.46 g, 3.23 mmol, 40% yield) as a pale yellow solid diastereomeric mixture of about 3:2. The collected low-purity Intermediate 1 (1.66 g) was purified by medium-pressure column chromatography on silica gel (10 g of arginine silica gel, acetonitrile/water = 100/0 to 97/3) to give Intermediate 1 (0.905 g, 2.00 mmol, 25% yield). The further collected low-purity Intermediate 1 (0.519 g) was purified by medium-pressure column chromatography on silica gel (10 g of arginine silica gel, acetonitrile/water = 100/0 to 97/3) to give Intermediate 1 (0.221 g, 0.489 mmol, 6% yield).
(1H NMR, LCMS: Chart 1-3)
1H NMR (270 MHz, DMSO-d6): δ 6.90-6.75 (m, 2H), 6.71 (s, 0.6H), 6.60-6.45 (m, 1.4H), 5.71 (s, 0.4H), 5.65 (s, 0.6H), 4.99 (t, 0.4H, J = 4.9 Hz), 4.82-4.60 (m, 1.6H), 3.90-3.48 (m, 14H), 3.48-3.18 (m, 7H), 3.18-2.75 (m, 4H), 2.17-1.86 (m, 2H).
LCMS (ESI+): m/z calcd for [M]+ = 417.1, RT: 1.05 min

### <Synthesis of JH013>

Fumaryl chloride (0.673 mL, 6.20 mmol) was dissolved in methylene chloride (10 mL), to which dried MS4A (1.40 g) was added and cooled to 0 °C in an ice bath. To this solution, a solution of Intermediate 1 (1.40 g, 3.10 mmol) in methylene chloride (18 mL) was added dropwise for 20 minutes, and then the mixture was stirred at room temperature for 2 hours. Thereafter, choline chloride (1.70 g, 12.2 mmol) was added and the mixture was further stirred at room temperature for 20 hours. The target substance precipitated as a viscous solid was collected through filtration, and the obtained solids were dissolved in a mixed solution of acetonitrile/methanol = 95/5 (50 mL), followed by removal of MS4A through filtration. The filtrate was carefully concentrated at about room temperature to obtain a crude product (3.33 g), which was then purified by medium-pressure column chromatography on silica gel (60 g of arginine silica gel, acetonitrile/water = 100/0 to 90/10). The eluate was directly freeze-dried to give JH013 (2.02 g) as a colorless solid comprising choline chloride and other substances. This JH013 was further purified by medium-pressure column chromatography on silica gel (60 g of ODS silica gel, acetonitrile/water = 1/99 to 5/95), and the eluate was directly freeze-dried to give JH013 (727 mg, 1.08 mmol, 35% yield) as a colorless solid diastereomeric mixture of about 7:3.
(1H NMR, LCMS: Chart 4-6)
1H NMR (270 MHz, DMSO-d6): δ 6.90-6.80 (m, 2.7H), 6.76-6.69 (m, 2H), 6.61-6.51 (m, 1.3H), 5.70 (s, 0.3H), 5.63 (s, 0.7H), 4.88-4.78 (m, 0.3H), 4.78-4.67 (m, 0.7H), 4.64-4.52 (m, 2H), 4.40-4.31 (m, 0.6H), 4.25-4.06 (m, 1.4H), 3.93-3.50 (m, 14H), 3.50-3.01 (m, 19H), 2.95-2.82 (m, 1H), 2.43-2.15 (m, 2H).
LCMS (ESI+): m/z calcd for [M]2+ = 300.7, RT: 1.02, 1.07 min

### Scheme of Synthesis of Compound JH014

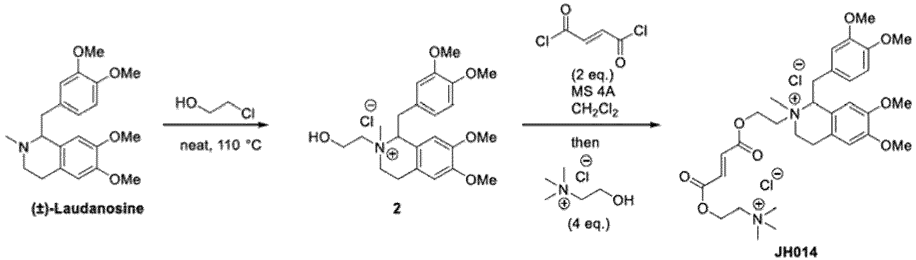

### <Synthesis of Intermediate 2>

To (±)-Laudanosine (8.22 g, 23.0 mmol) was added ethylene chlorohydrin (7.72 mL, 115 mmol), and the mixture was heated at 110°C for 14 hours. After leaving the mixture to cool, acetonitrile (15 mL) was added to completely dissolve the precipitated target substance. Then, the reaction solution was added dropwise to diisopropyl ether (300 mL), and the precipitated solids were collected through filtration. The same operation was repeated twice for the resulting solids received. The resulting crude product (11.6 g) was purified by medium-pressure column chromatography on silica gel (100 g of diol silica gel, methylene chloride/methanol = 100/0 to 95/5) to obtain Intermediate 2 (8.52 g) as a mixture. This mixture was divided into two parts, each of which was then purified by medium-pressure column chromatography on silica gel (60 g of arginine silica gel, acetonitrile/water = 97/3) to give Intermediate 2 (6.33 g, 14.5 mmol, 63% yield) as a pale yellow solid diastereomeric mixture of about 7:3. The low-purity Intermediate 2 (1.29 g) collected in this step was further purified by medium-pressure column chromatography on silica gel (30 g of arginine silica gel, acetonitrile/water = 100/0 to 97/3) to give Intermediate 2 (0.780 g, 1.78 mmol, 8% yield).
(1H NMR, LCMS: Chart 7-9)
1H NMR (270 MHz, DMSO-d6): δ 6.90-6.78 (m, 2H), 6.70 (s, 0.7H), 6.65 (s, 0.3H), 6.58-6.51 (m, 0.7H), 6.51-6.44 (m, 0.3H), 6.58-6.44 (m, 1H), 5.71 (t, 0.3H, J = 4.9 Hz), 5.64 (s, 1H), 5.48 (t, 0.7H, J = 4.9 Hz), 4.95-4.77 (m, 1H), 4.15-4.00 (m, 0.6H), 3.95-3.54 (m, 13.4H), 3.43-3.27 (m, 4H), 3.27-3.20 (m, 3H), 3.17-3.03 (m, 3H), 2.92-2.72 (m, 1H).
LCMS (ESI+): m/z calcd for [M]+ = 403.1, RT: 0.93 min

### <Synthesis of JH014>

Fumaryl chloride (0.897 mL, 8.27 mmol) was dissolved in methylene chloride (22.5 mL), to which dried MS4A (1.81 g) was added and cooled to 0°C in an ice bath. To this solution a solution of Intermediate 2 (1.81 g, 4.13 mmol) in methylene chloride (10 mL) was added dropwise for 10 minutes, and then the mixture was stirred at room temperature for 5 hours. Thereafter, choline chloride (2.31 g, 16.5 mmol) was added and the mixture was further stirred at room temperature for 61 hours. The target substance precipitated as a viscous solid was collected through filtration, and the obtained solids were dissolved in a mixed solution of acetonitrile/methanol = 95/5 (100 mL), followed by removal of MS4A through filtration. The filtrate was carefully concentrated at about room temperature to obtain a crude product (3.87 g), which was then purified by medium-pressure column chromatography on silica gel (90 g of arginine silica gel, acetonitrile/water = 100/0 to 93/7). The eluate was directly freeze-dried to give JH014 (927 mg) as a colorless solid comprising choline chloride and other substances. This JH014 was further purified by medium-pressure column chromatography on silica gel (60 g of ODS silica gel, acetonitrile/water = 1/99 to 5/95), and the eluate was directly freeze-dried to give JH014 (586 mg, 0.891 mmol, 22% yield) as a colorless solid diastereomeric mixture of about 3:1.
(1H NMR, LCMS: Chart 10-12)
1H NMR (270 MHz, DMSO-d6): δ6.94-6.74 (m, 4.75H), 6.65 (s, 0.25H), 6.57-6.50 (m, 1H), 5.71 (s, 0.25H), 5.57 (s, 0.75H), 5.05-4.97 (m, 0.25H), 4.88-4.78 (m, 1.25H), 4.72-4.55 (m, 3.5H), 4.00-3.55 (m, 14H), 3.50-3.05 (m, 19H), 2.93-2.80 (m, 1H).
LCMS (ESI+): m/z calcd for [M]2+ = 293.7, RT: 1.00, 1.05 min

### <Evaluation of Neuromuscular Blocking Effect>

Male Sprague Dawley rats (about 300 g body weight) were used in the experiment. Anesthesia was first induced by 4 to 5% isoflurane inhalation and then maintained by 2 to 3% isoflurane inhalation. A warming system was used to keep the body temperature at 36 to 37°C. Lidocaine was infiltrated into the incision site. Tracheostomy was performed and a 16G catheter was inserted to secure the airway. The animals were ventilated by isoflurane and oxygen inhalation using a rodent ventilator at a tidal volume of 10 mL/kg and a respiratory rate of 70/min. A 24G catheter was indwelled in the right jugular vein for drug administration. A small slide electrode was attached to the sciatic nerve, and supramaximal stimulation was applied at a frequency of 0.1 Hz and a pulse width of 0.2 ms using an electrostimulator. Using a force transducer, the twitch response of the gastrocnemius muscle was captured, amplified, and recorded. After the amplitude of the twitch response stabilized, a dose of succinylcholine or the JH-cpd series (JH013, JH014) inducing 95-99.9% neuromuscular block was administered, and the time until the muscle contraction (twitch response) was completely recovered was measured.

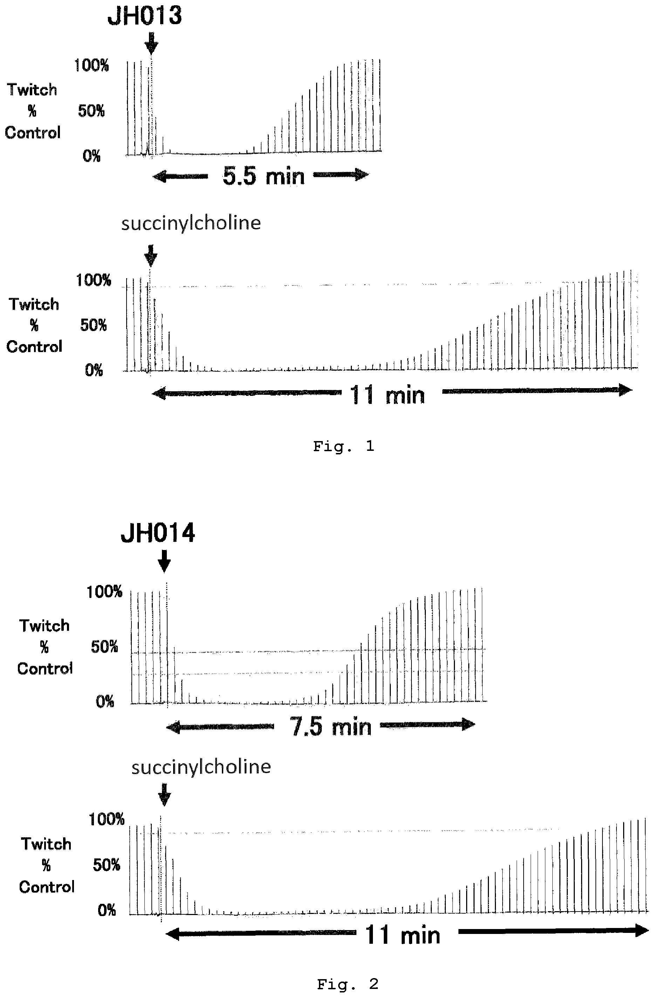

As a result, the time until the muscle contraction (twitch response) was completely recovered was 11 minutes for succinylcholine, whereas it was 5.5 minutes for JH013 (Figure 1) and 7.5 minutes for JH014 (Figure 2). In other words, it can be said that JH013 successfully shortened the duration of action of succinylcholine to approximately half, and JH014 to approximately two-thirds.

## Claims

1. A compound represented by the general formula (I), or a pharmacologically acceptable salt thereof, 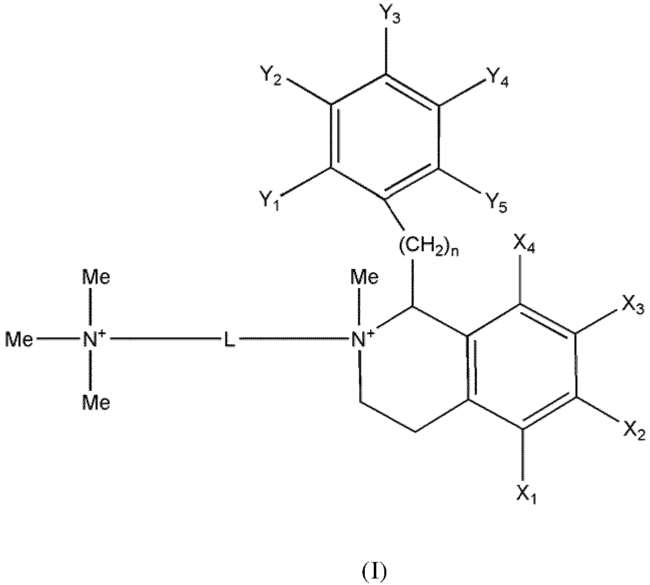 wherein, in the general formula (I),
X₁, X₂, X₃, and X₄ each independently represent hydrogen or a C₁₋₃ alkoxy group, and at least two of X₁, X₂, X₃, and X₄ are C₁₋₃ alkoxy groups,
Y₁, Y₂, Y₃, Y₄, and Y₅ each independently represent hydrogen or a C₁₋₃ alkoxy group, and at least two of Y₁, Y₂, Y₃, Y₄, and Y₅ are C₁₋₃ alkoxy groups,
n is 0 or 1, and
L represents a linker with a main chain of 5 to 20 atoms.

2. The compound or a pharmacologically acceptable salt thereof according to claim 1, wherein
X₁ and X₄ are hydrogen, and X₂ and X₃ are C₁₋₃ alkoxy groups, and
Y₃ and either Y₂ or Y₄ of Y₁, Y₂, Y₃, Y₄, and Y₅ are C₁₋₃ alkoxy groups, and the remaining groups are hydrogen.

3. The compound or a pharmacologically acceptable salt thereof according to claim 1 or 2, wherein
L is a divalent alkylene or alkenylene group with a main chain of 5 to 20 carbon atoms, wherein one or more -CH₂- is replaced by -O- or -O-C(=O)- or -C(=O)-O-, and one or more hydrogen is optionally replaced by halogen.

4. The compound or a pharmacologically acceptable salt thereof according to any one of claims 1 to 3, wherein
L is selected from the following general formulae (II) to (V), 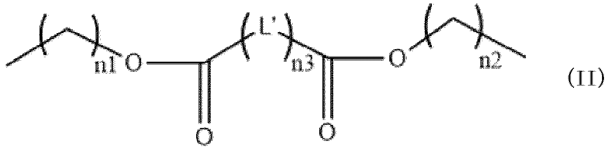 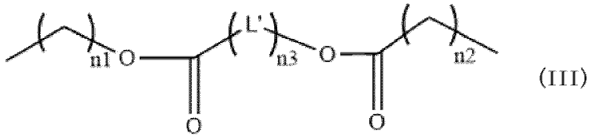 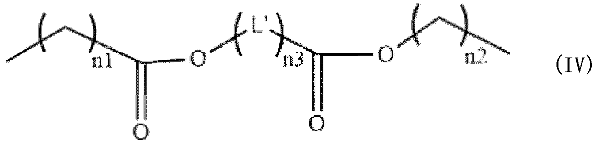 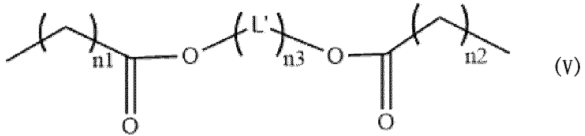 wherein,
-(L')ₙ₃- is a C₁₋₆ alkylene or alkenylene group wherein one or more hydrogen is optionally replaced by halogen,
n1 is an integer of 1 to 5, and
n2 is an integer of 1 to 5.

5. The compound or a pharmacologically acceptable salt thereof according to any one of claims 1 to 3, wherein
L is represented by the general formula (VI), 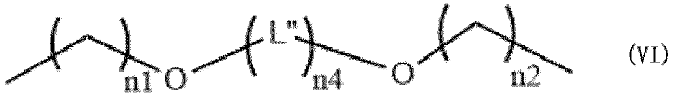 wherein,
-(L")ₙ₄- is a C₁₋₆ alkylene or alkenylene group wherein one or more hydrogen is optionally replaced by halogen,
n1 is an integer of 1 to 5, and
n2 is an integer of 1 to 5.

6. The compound or a pharmacologically acceptable salt thereof according to claim 4, wherein
the compound represented by the general formula (I) is selected from the following compounds.

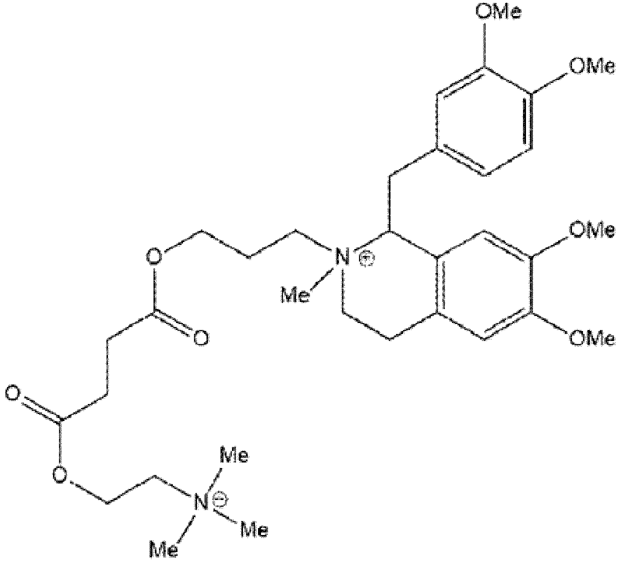

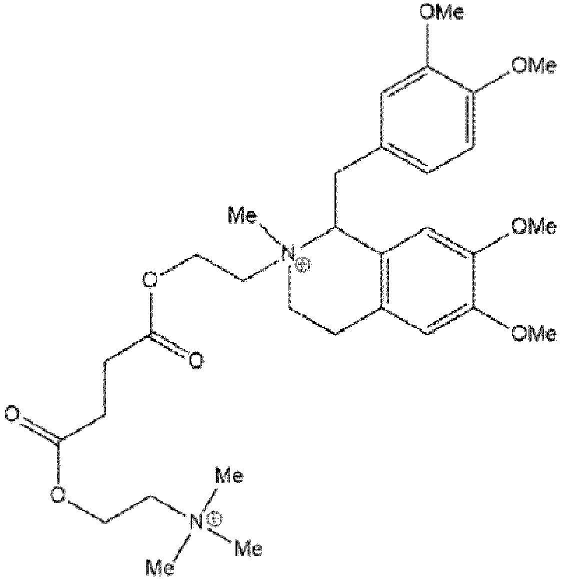

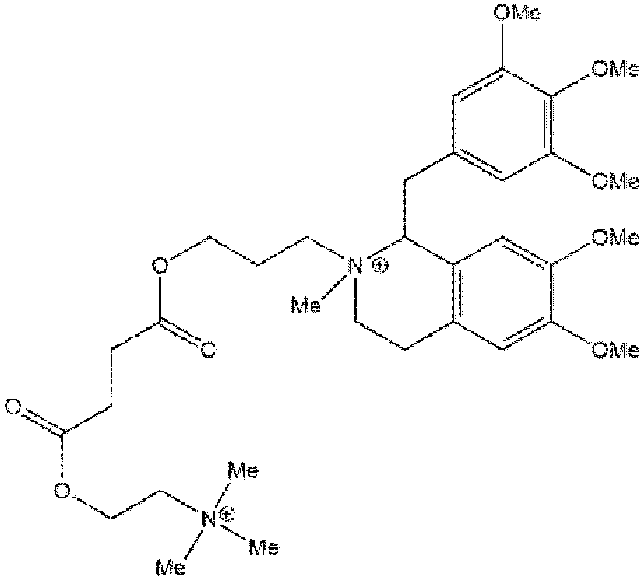

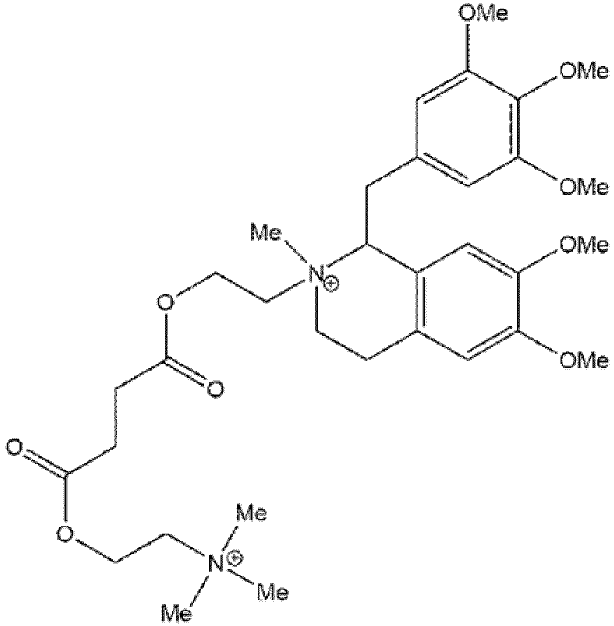

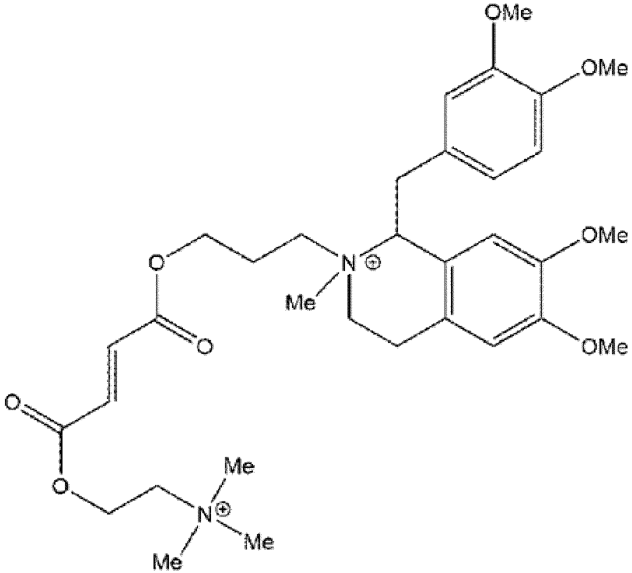

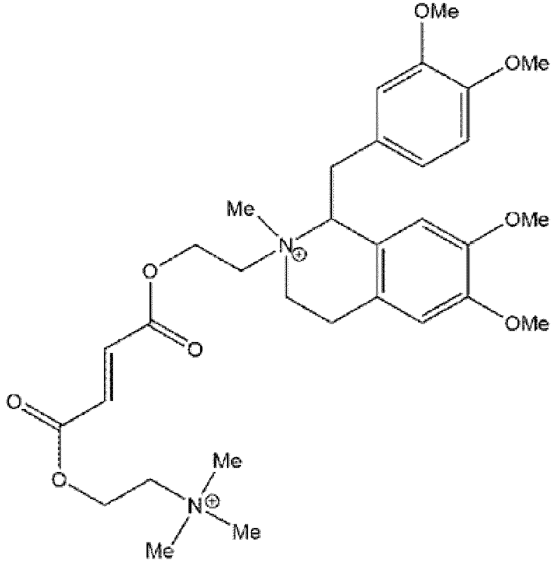

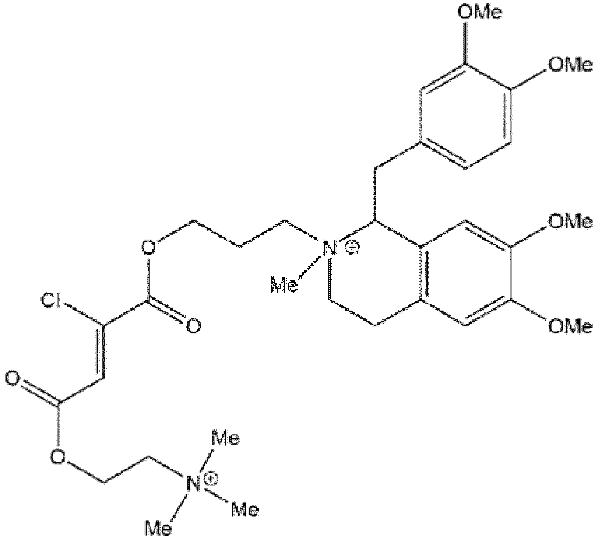

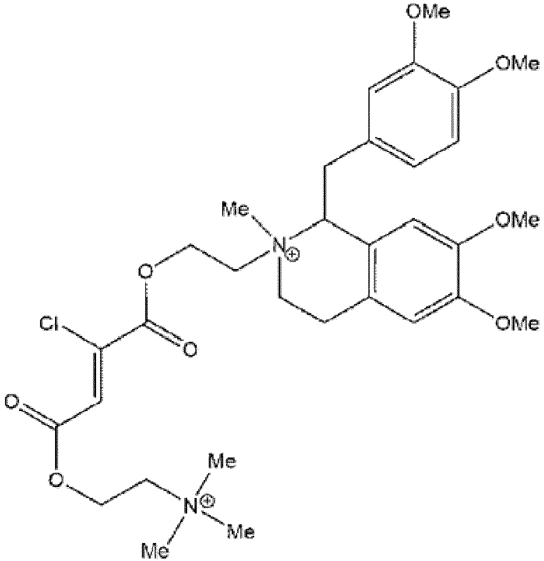

7. A pharmaceutical composition comprising the compound or a pharmacologically acceptable salt thereof according to any one of claims 1 to 6, and a pharmacologically acceptable carrier.

8. The pharmaceutical composition of claim 7, for use as a neuromuscular blocking agent.
